# EUROPÄISCHE PATENTSCHRIFT

(11) **EP 0 669 139 B1**
(45) Veröffentlichungstag und Bekanntmachung des Hinweises auf die Patenterteilung: **27.05.1998**
(21) Anmeldenummer: 95102533.7
(22) Anmeldetag: 22.02.1995
(51) Int. Cl.: A61M 1/00

(54) **Vorrichtung zum Sammeln und groben Reinigen von bei Operationen an der Operationsstelle anfallendem Blut**
Device for recovering and coarse filtration of blood from an operating theatre
Dispositif de récupération et de filtration grossière du sang provenant d'un champ opératoire

(30) Priorität: 26.02.1994 DE 9403245 U
(43) Veröffentlichungstag der Anmeldung: 30.08.1995
(73) Patentinhaber: Schneider, Lothar, 77866 Rheinau-Honau (DE)
(72) Erfinder: Schneider, Lothar, 77866 Rheinau-Honau (DE)
(74) Vertreter: Zipse + Habersack

(56) Entgegenhaltungen:
- EP-A- 0 525 493
- WO-A-91/12033
- DE-U- 9 403 245
- US-A- 4 111 204
- US-A- 4 306 557
- US-A- 4 772 256

## Beschreibung

Die Erfindung betrifft eine Vorrichtung zum Sammeln und groben Reinigen von bei Operationen an der Operationsstelle anfallendem Blut, das mittels eines nachfolgend installierten Aufbereitungsgerätes dem Patienten während der Operation wieder zur Verfügung gestellt wird, mit einem im wesentlichen zylindrischen Behälter, auf dem ein Deckel befestigbar ist, der mit einem in den Behälter reichenden, dehnbaren Aufnahmemedium für das Blut verbunden ist, wobei am Deckel ein Filtersieb und mindestens ein Anschluß für einen Absaugschlauch für das Blut angeordnet ist.

Bei Operationen kommt es sehr häufig vor, daß an der Operationsstelle, beispielsweise der Wunde, sehr viel Blut anfällt, das bisher abgesaugt und verworfen wurde. Da dieses Blut aber im Hinblick auf die Versorgung des Patienten mit Spenderblut eine sehr wertvolle Bereicherung und Ergänzung sein kann, wurde schon vorgeschlagen, das Blut abzusaugen und wieder so auf zubereiten, daß es dem Patienten wieder zugeführt werden kann.

In der EP-A-0 345 831 ist ein Autotransfusionsgerät zum Sammeln von Blut und anderen Körperflüssigkeiten beschrieben, das aus einem festen, unterdruckstabilen Behälter besteht, der über eine Auslaßöffnung im Deckelteil oder eine Öffnung im Bodenteil evakuierbar ist. Im Behälter ist am oberen Rand eine Membran befestigt, die sich an die Innenkontur des Deckelteiles sowie, alternierend, an die Innenkontur des Bodenteils im wesentlichen anlegt. Dabei kann die Verbindung zwischen dem Membranrand und dem Boden- und Deckelteil durch Kunststoffverschweißen unter Bildung eines einteiligen Einmalbehälters erfolgen. Bei völlig mit Blut gefüllten Behältern nimmt die Membran im wesentlichen die Form der Innenkontur an. Bei einer besonderen Ausführungsform des vorbekannten Autotransfusionsgerätes ist auf der Seite des Behälters ein Anschluß für den Unter- oder Überdruck direkt im Behälter vorgesehen. Zum mehrfachen Gebrauch müssen die Teile des vorbekannten Autotransfusionsgerätes nach Gebrauch zerlegt und wieder gereinigt werden. Dies ist verhältnismäßig aufwendig.

Der vorliegenden Erfindung liegt daher die Aufgabe zugrunde, eine Vorrichtung der eingangs genannten Art zu schaffen, mit der in einfacher Weise das Blut abgesaugt, gesammelt und schließlich von groben Partikeln gereinigt werden kann, um es mittels eines nachfolgend installierten Aufbereitungsgerätes dem Patienten während der Operation wieder zur Verfügung zu stellen, wobei es im wesentlichen darauf ankommt, die komplizierten Teile im Einmalbetrieb zu verwenden, so daß sich die Reinigungsvorgänge nur auf einen zylindrischen Behälter beziehen, während die anderen Teile nach dem Gebrauch weggeworfen werden.

Zur Lösung der gestellten Aufgabe wird eine Vorrichtung gemäß Patentanspruch 1 vorgeschlagen.

Die Vorrichtung gemäß der vorliegenden Erfindung weist den Vorteil auf, daß die wesentlichen Teile mit dem transportablen Kunststoffbeutel integriert sind, so daß sie im Einmalbetrieb verwendet werden können. Außerdem ist bei der vorliegenden Erfindung lediglich ein einfacher zylindrischer Behälter zu reinigen, während der Deckel mit dem Kunststoffbeutel als Blutaufnahmemedium nach der Benutzung verworfen wird.

Ein weiterer wesentlicher Unterschied der vorliegenden Erfindung gegenüber dem bekannten Autotransfusionsgerät besteht darin, daß bei der vorbekannten Vorrichtung das Blut wieder durch einen Gegendruck in den Behälter aus der Membran herausgedrückt wird.

In den Unteransprüchen sind wertvolle Bereicherungen der Vorrichtung gemäß der Erfindung dargestellt.

So kann gemaß Anspruch 2 der Deckel an der Unterseite mit einem O-förmigen Dichtungsring versehen sein und mittels eines Verschlusses, beispielsweise eines Bajonettverschlusses, dicht mit dem Behälter verbunden werden.

Gemäß Anspruch 3 reicht der Beutel bis zum Boden des Behälters und ist an der Unterseite schräg verschweißt, damit das anfallende Blut vollständig abgesaugt werden kann.

Der Behälter kann ferner autoklavierbar ausgebildet sein und aus Polycarbonat bestehen.

Anhand der Zeichnung soll am Beispiel einer bevorzugten Ausführungsform die Vorrichtung gemäß der Erfindung näher erläutert werden.

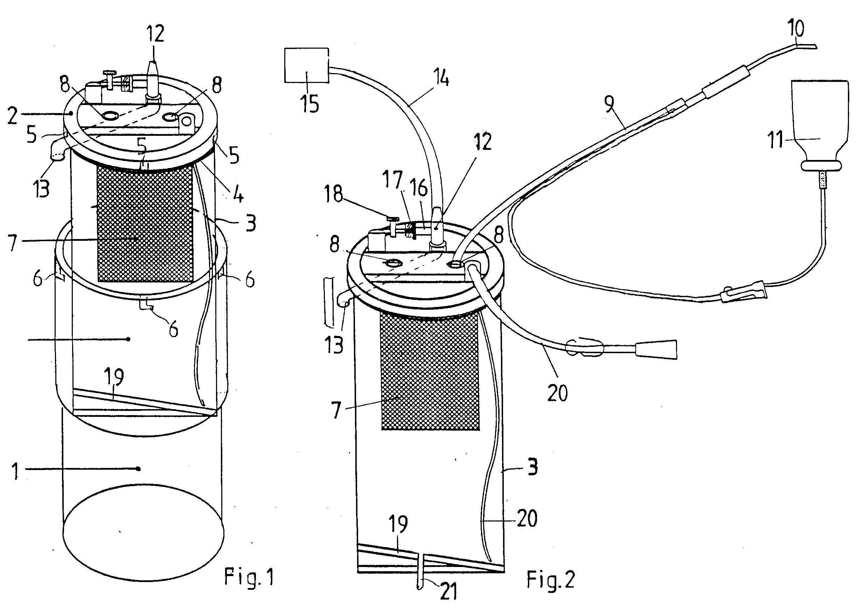

In der Zeichnung zeigt
- Fig. 1: eine Prinzipdarstellung des Behälters mit teilweise eingesetztem Deckel.
- Fig. 2: zeigt den Deckel und den damit verbundenen Blutauffangbeutel sowie eine angeschlossene Absaugvorrichtung.

Wie sich aus den Figuren der Zeichnung ergibt, besteht die Vorrichtung gemäß ein bevorzugtes Beispiel der Erfindung aus einem im wesentlichen zylindrischen Behälter 1 aus Polycarbonat, der so ausgebildet ist, daß er in einem Autoklaven sterilisiert werden kann. Der Behälter ist mittig so verstärkt, daß diese Verstärkung zur Aufnahme an einen Infusionsständer dient. Auf diesen Behälter 1 kann ein Deckel 2 aufgesetzt und fest mit ihm verbunden werden, der an der Unterseite einen etwas zurück versetzten Kunststoffbeutel 3 zum Auffangen des Blutes aufweist. Der Deckel 2 weist hierzu an der Unterseite einen O-förmigen Dichtungsring 4 auf und kann mittels eines Verschlusses, beispielsweise eines Bajonettverschlusses 5, 6, auf dem Behälter festgelegt werden. Der Kunststoffbeutel 3 befindet sich dann vollständig im Inneren des Behälters 1.

An der Unterseite des Deckels 2 ist ferner ein in den Beutel 3 reichendes Filtersieb 7 angeordnet, über dem sich zwei Anschlüsse 8 befinden, wovon der eine verschlossen und der zweite mit einem doppellumigen Absaugschlauch 9 verbunden ist, der zu einer Absaugsonde 10 führt, die an die Stelle des Blutaustritts in der Wunde gebracht wird. Mittels eines Infusionsgerätes 11 können noch bestimmte Stoffe, z. B. Heparin, dem Blut zugeführt werden.

Im Deckel 2 ist ferner ein Ansaugstutzen 12 zur Erzeugung des Vakuums vorgesehen, der mit einem im Deckel verlaufenden Kanal 13 verbunden ist, der in dem Raum zwischen der Außenseite des Beutels 3 und der Innenseite des Behälters 1 endet. Der Ansaugstutzen 12 ist über einen Schlauch 14 mit der krankenhausinternen Druckluftanlage 15 verbunden, die durch ein Venturi-System ein Vakuum erzeugt. Durch die dargestellte Anordnung wird verhindert, daß der Beutel 3 während des Absaugen des Blutes durch das Vakuum zusammenfällt.

Der Ansaugstutzen 12 weist zur Erzeugung des Vakuums im Inneren des Beutels 3 einen Abgang 16 auf, der über ein Filter 17 und einen Zweiwegehahn 18 mit dem Innenraum des Beutels 3 in Verbindung steht.

Der Beutel 3 weist an der Unterseite eine schräge Verschweißung 19 auf. In den Beutel 3 reicht ferner ein Schlauch 20 bis an die tiefste Stelle, der durch den Deckel 2 geführt wird und mit seinem äußeren Ende bis zum Blutaufbereitungsgerät reicht.

Bei der dargestellten Ausführungsform ist die Membran des Filters 17 hydrophob ausgebildet. Dieses System ermöglicht es, Luftmengen, die zusammen mit dem Blut durch den doppellumigen Schlauch 9 in den Beutel 3 gelangen, über die Vakuumabsaugung am Anschluß 12 zu eliminieren. Das Filter 17 schützt den Schlauch 14 und das Vakuumsystem 15 vor Verunreinigung.

Das Fassungsvermögen des Behälters 1 beträgt etwa 3.000 ml. Das Filtersieb 7 hat eine Maschenweite von 170 micron.

An der Unterseite der schrägen Verschweißung 19 des Beutels 3 kann eine Ablaufvorrichtung 21 vorgesehen sein, die zum Entleeren des Blutes aus dem Beutel mittels Schwerkraft dient.

## Patentansprüche

1. Vorrichtung zum Sammeln und groben Reinigen von bei Operationen an der Operationsstelle anfallendem Blut, das mittels eines nachfolgend installierten Aufbereitungsgerätes dem Patienten während der Operation wieder zur Verfügung gestellt wird, mit einem im wesentlichen zylindrischen Behälter (1) mit einem darauf abnehmbar angeordneten Deckel (2), der mit einem in den Behälter (1) reichenden, dehnbaren Aufnahmemedium (3) für das Blut verbunden ist, wobei am Deckel (2) ein Filtersieb (7) und mindestens ein Anschluß für einen Absaugschlauch (9) für das Blut angeordnet ist, ***dadurch gekennzeichnet,*** daß das dehnbare Aufnahmemedium für das Blut aus einem mit dem Deckel verbundenen und mit diesem eine vom Behälter abnehmbare Einheit bildenden Kunststoffbeutel (3) zur Aufbewahrung und zum vorübergehenden Transport des Blutes besteht, in welchen Kunststoffbeutel das Filtersieb (7) reicht, und daß im Deckel (2) ein Ansaugstutzen (12) zur Erzeugung eines Vakuums angeordnet ist, der mit einem im Deckel verlaufenden Kanal (13) verbunden ist, der in dem Raum zwischen der Außenseite des Beutels (3) und der Innenseite des Behälters (1) endet, wobei der Ansaugstutzen (12) oberhalb des Deckels (2) mit einem Abgang (16) versehen ist, der über ein hydrophobes Filter (17) und einen Zweiwegehahn (18) mit dem Innenraum des Beutels in Verbindung steht, und daß bis an die tiefste Stelle des Beutels (3) ein durch den Deckel (2) geführter und zum Aufbereitungsgerät reichender Schlauch (20) geführt ist.

2. Vorrichtung nach Anspruch 1, ***dadurch gekennzeichnet,*** daß der Deckel (2) an der Unterseite mit einem O-förmigen Dichtungsring (4) versehen ist und mittels eines Verschlusses, beispielsweise eines Bajonettverschlusses (5, 6), mit dem Behälter dicht verbindbar ist.

3. Vorrichtung nach Anspruch 1 oder 2, ***dadurch gekennzeichnet,*** daß der Beutel bis zum Boden des Behälters (1) reicht und an der Unterseite eine schräge Verschweißung (19) aufweist.

4. Vorrichtung nach einem oder mehreren der vorhergehenden Ansprüche, ***dadurch gekennzeichnet,*** daß der Behälter (1) autoklavierbar ausgebildet ist und aus Polycarbonat besteht.

5. Vorrichtung nach Anspruch 3, ***dadurch gekennzeichnet,*** daß an der Unterseite der schrägen Verschweißung (19) des Beutels (3) eine Ablaufvorrichtung (21) vorgesehen ist, die zum Entleeren des Blutes aus dem Beutel mittels Schwerkraft dient.

## Claims

1. Device for the collection and coarse cleansing of blood which accumulates in the course of operations at the site of the operation and which is made available again to the patient during the operation by means of a subsequently installed refining apparatus, comprising a substantially cylindrical container (1) with a removable cover (2) disposed thereon that is connected to an expandable receiving medium (3) for the blood which extends into the container (1), whereby a filtering sieve (7) and at least one port for a suction tube (9) for the blood are disposed on the cover (2), ***characterised in that*** the expandable receiving medium for the blood consists of a plastic bag (3) for the storage and temporary transport of the blood, said plastic bag being connected to the cover and forming with the latter a unit that can be removed from the container, whereby the filtering sieve (7) extends into said plastic bag, and in that a suction port (12) for generating a vacuum is disposed within the cover (2), said suction port being connected to a channel (13) located within the cover which terminates in the space between the outside of the bag (3) and the inside of the container (1), whereby the suction port (12) is provided above the cover (2) with an outlet (16) which is connected via a hydrophobic filter (17) and a two-way stopcock (18) to the interior space of the bag, and in that a tube (20) which is guided through the cover (2) and which extends to the refining apparatus is guided as far as the lowest point of the bag (3).

2. Device according to Claim 1, ***characterised in that*** the cover (2) is provided on the underside with an O-shaped sealing ring (4) and is capable of being tightly connected to the container by means of a lock, for example by means of a bayonet lock (5, 6).

3. Device according to Claim 1 or 2, ***characterised in that*** the bag extends as far as the bottom of the container (1) and has an oblique heat seal (19) on the underside.

4. Device according to one or more of the preceding claims, ***characterised in that*** the container (1) is designed to be autoclavable and consists of polycarbonate.

5. Device according to Claim 3, ***characterised in that*** a drainage device (21) is provided on the underside of the oblique heat seal (19) of the bag (3), said drainage device serving to empty the blood from the bag by means of gravity.

## Revendications

1. Dispositif de collecte et d'épuration grossière de sang répandu au point d'opération lors d'interventions chirurgicales et remis à la disposition du patient, pendant l'opération, au moyen d'un appareil de préparation installé à la suite du dispositif, lequel comprend un récipient (1) essentiellement cylindrique avec un couvercle (2) disposé amovible sur lui et relié à un milieu récepteur (3) pour le sang, qui s'étend à l'intérieur du récipient et est extensible, avec disposition sur le couvercle (2) d'un tamis filtrant (7) et d'au moins un raccord pour un tuyau souple d'aspiration (9) pour le sang, **caractérisé en ce** que le milieu récepteur extensible pour le sang est constitué d'une poche (3) de matière plastique pour la conservation et le transport temporaire du sang, qui est reliée au couvercle et forme avec celui-ci un ensemble pouvant être retiré du récipient, le tamis filtrant (7) s'étendant à l'intérieur de cette poche de matière plastique, qu'une tubulure d'aspiration (12) pour créer un vide est disposée dans le couvercle (2) et reliée à un canal (13) s'étendant dans le couvercle et se terminant dans l'espace situé entre le côté extérieur de la poche (3) et le côté intérieur du récipient (1), la tubulure d'aspiration (12) étant munie, au-dessus du couvercle (2), d'un départ (16) qui communique à travers un filtre hydrophobe (17) et un robinet à deux voies (18) avec l'intérieur de la poche, et qu'un tuyau souple (20) s'étend jusqu'au point le plus bas de la poche (3), traverse le couvercle (2) et mène à l'appareil de préparation.

2. Dispositif selon la revendication 1, caractérisé en ce que le couvercle (2) est pourvu, sur son dessous, d'un joint torique d'étanchéité (4) et peut être fixé de façon étanche au récipient au moyen d'un assemblage, par exemple d'un joint à baïonnette (5, 6).

3. Dispositif selon la revendication 1 ou 2, caractérisé en ce que la poche s'étend jusqu'au fond du récipient (1) et présente sur son côté inférieur une soudure oblique (19).

4. Dispositif selon une ou plusieurs des revendications précédentes, caractérisé en ce que le récipient (1) est réalisé en vue d'un traitement en autoclave et est fabriqué de polycarbonate.

5. Dispositif selon la revendication 3, caractérisé en ce qu'un dispositif d'écoulement (21) est prévu sur le côté inférieur de la soudure oblique (19) de la poche (3) en vue du vidage de la poche de son sang par gravité.
